# EUROPEAN PATENT SPECIFICATION

(11) **EP 0 743 060 B1**
(45) Date of publication and mention of the grant of the patent: **13.11.2002**
(21) Application number: 95201116.1
(22) Date of filing: 28.04.1995
(51) Int. Cl.: A61K 7/26, A61K 35/20

(54) **Use of dental-care products comprising bovine colostrum**
Verwendung von Zahnpflegemitteln enthaltend bovines Kolostrum
Utilisation de produits d'hygiène dentaire comprenant du colostrum bovin

(43) Date of publication of application: 20.11.1996
(73) Proprietor: Sara Lee/DE N.V., 3532 AA Utrecht (NL)
(72) Inventor: Timmer, Christiena Jannie, 3811 WK Amersfoort (DE)
(74) Representative: Smulders, Theodorus A.H.J., Ir.

(56) References cited:
- FR-A- 1 580 913
- GB-A- 1 505 513
- GB-A- 2 052 979
- S.T.N., File Supplier, Karlsruhe, DE,
- S.T.N., File Supplier, Karlsruhe, DE

## Description

The present invention is related to mouth-care products having improved properties, especially in relation to counteracting various irritations and/or diseases of the oral cavity.

Mouth-care products are defined as products that are intended to be used in the oral cavity for some time having a cleaning and/or healing and/or prophylactic function, but which are not intended to be swallowed.

In the development of mouth-care products much attention has been paid to various aspects of oral hygiene and oral care. The reduction of the number of bacteria in the oral cavity, the treatment of various mucous disorders and the prophylactic (preventive) function of oral care products has been subject of much research and development.

Bacteria that are present in the oral cavity lead to dental plaque formation. Plaque is considered to be the prime etiological factor in the development of caries, and it is also implicated in periodontal diseases. Plaque, when not sufficiently removed, develops into calculus, a hard, mineralised formation which deposits on the enamel surface of the teeth. There is also an association between the presence of calculus and the incidence of periodontal diseases. Serious teeth problems, like caries, gingivitis and other periodontal diseases, can be the result of formation of plaque and calculus.

In the prior art many different lines of approach have been used to find solutions to the problems in the field of oral care.

Regular brushing is the most used method for prevention of build-up of plaque, but even regular brushing is not sufficient to remove all of the plaque which adheres to the teeth. Plaque is formed rather fast and accordingly toothpastes have been proposed that prevent rapid build-up of plaque or that remove plaque chemically.

Antiplaque dentifrice's, based on special chemical compounds, have been described. Examples of these special ingredients are quaternary ammonium compounds, described in US 4 323 551, sanquinarine (GB 2 042 336), Triclosan (5-chloro-2-(2,4-dichlorophenoxy)phenol) described in EP 0 161 899, p-aminobenzoic acid (US 3 427 380) and many others.

As plaque is developed by bacteria in the oral cavity, is has been proposed to use bactericides as antiplaque agents. The most important ingredient is chlorhexidine, described in many patents, for example DE 1 084 876. A disadvantage of this compound is the discoloration of the teeth. Compounds like bis-biguanides (US 4 067 962) and alexidine (EP 0 004 719) did not stain the teeth, but proved to be insufficiently effective too.

However, a reduction of the bacterial activity in the oral cavity is most important, as this activity is a major cause of most problems related to oral hygienic conditions.

It has already been proposed that plaque can be effectively be prevented by addition of specific bacteriocin compounds, such as lantibiotics, i.e nisin, subtilin and comparable compounds, in combination with at least one compound yielding fluoride ions, more in particular an alkali metal fluoride, to mouth-care products used to take care of the teeth and oral health.

Other aspects of oral care concern the preventive aspects thereof, such as strengthening the immuno-system of the oral cavity by supplying compounds having this action. In addition thereto it is of interest to provide oral care products having a healing funtion, such as in the treatment of contact lesions, burns, irritations, aphtous ulceration, mucositis, and the like. Also the prevention and treatment of allergic disorders, inflammations ageing and the like is of importance.

The object of the present invention is to provide an oral care product that is able to provide and/or strengthen the antibacterial activity in the oral cavity and/or is able to provide the other functions described above. More in particular the invention provides for an oral care product that strengthens the natural defence system in the mouth, resulting in an improved healing of disorder of the gums.

The present invention is based on the surprising discovery, that colostrum and more in particular bovine colostrum, when incorporated in an oral care product is able to provide the required functions. It is especially noted that the presence of colostrum in the oral care product strengthens the natural defence system and results in improved healing of gums.

The present invention is therefore directed to a mouth-care product comprising an effective amount of colostrum and/or an extract thereof.

The present invention is also directed to a method to prevent the accumulation of dental plaque, thus reducing the formation of calculus and occurrence of caries, gingivitis, mucositis and/or other periodontal diseases.

In a third aspect the present invention is directed to the use of colostrum and/or an extract thereof, in mouth-care products to prevent the accumulation of dental plaque, thus reducing the formation of calculus and occurrence of caries, gingivitis and/or other periodontal diseases.

Colostrum to be used in the mouth- or oral-care products of the present invention consist of a mixture of lacteal secretions and constituents of blood serum, notably immunoglobulins and other proteins, that accumulate in the mammary gland during the prepartum dry period and can be harvested immediately preceding or following parturition. The first six milkings collected during the period of transition from colostrum to milk are typically referred to as colostrum, in the case of cows as bovine colostrum. More in particular colostrum is generally defined in the art as the mammary secretion occurring in the period starting some days before delivery until some days after delivery.

In the present invention either colostrum itself, preferably in dry form, such as lyophilised, or spray dried, or in the form of an extract, such as a concentrate or purfied form, may be used.

The colostrum used in the present invention can be from any source, such as cows, sheep, goats and the like, but it is preferred to use bovine colostrum as this has been found to be particularly effective.

According to the invention an effective amount of colostrum and/or an extract of colostrum is present in a mouth-care product. An effective amount refers to an amount that performs an antibacterial and/or antimicrobial activity in the oral cavity and/or the other healing and/or prophylactic properties during application of the mouth-care product according to the present invention. Such activity will in general result if a total amount of between 0.1 and 50 wt.%, preferably an amount of between 0.5 and 25 wt.% of colostrum, calculated on the dry weight of the product is added to the mouth-care product. Preferably the upper total amount does not exceed 90 wt.%.

For various types of mouth-care products different ranges may be used. The general range and the preferred amount in weight % is given in the following list:

| | | |
|---|---|---|
| Toothpaste | 1-50 | 10 |
| Mouth water | 0.1-5 | 0.5 |
| Mouth rinse | 1-10 | 4 |
| Chewing tablet | 1-10 | 2 |
| Effervescent tablet | 5-25 | 10 |
| Tooth powder | 5-50 | 15 |

Colostrum and more in particular bovine colostrum is known to contain an increased amount of compounds that are active in preventing various disorders in the new-born calves. More in particular colostrum contains, compared to regular whole milk, an increased amount of immunoglobulins, such as IgG, IgM and IgA. Especially the increased amount of IgG is of importance in the oral care products. It is to be noted that using a purified form of colostrum containing a further increased amount of IgG is of especial importance.

The presence of fluoride providing agents in the mouth-care products according to the present invention is important. The use of fluoride ions, or fluor-ion providing compounds as anti-caries agents is well known in the art. Suitable compounds may be slightly soluble in water or may be fully water-soluble. They are characterized by their ability to release fluoride ions in water and by freedom from undesired reaction with other compounds of the oral preparation. Especially the use of alkalimetal fluorides, more in particular sodium fluoride, results in improved activity of the oral care products.

Among suitable compounds yielding fluoride ions are inorganic fluoride salts, such as soluble alkali metal and alkaline earth metal salts, for example sodium fluoride, potassium fluoride, barium fluoride, or calcium fluoride and ammonium fluoride, or a copper fluoride, or zinc fluoride, sodium fluorosilicate, ammonium fluorosilicate, sodium fluorozirconate, sodium mono-fluorophosphate, aluminium mono- and di-fluorophosphate, and fluorinated sodium calcium pyrophosphate. Alkali metal and tin fluorides, such as sodium and stannous fluorides, sodium monofluorophosphate, organic fluorides, such as aminfluoride and mixtures thereof, are preferred.

The amount of fluoride-providing compounds is dependent to some extent upon the type of compound, its solubility, and the type of oral preparation, but it must be a nontoxic, effective amount, generally about 0.005 to about 3.0% in the preparation, e.g. gel, tooth paste or tooth powder, an amount of such compound which releases up to about 5,000 ppm of F-ion by weight of the preparation is considered satisfactory. Any suitable minimum amount of such compound may be used, but it is preferable to employ sufficient compound to release about 300 to about 2,000 ppm, more preferably about 800 to about 1,500 ppm of fluoride ion. Typically, in the case of alkali metal fluorides and stannous fluoride, this component is present in an amount up to about 2% by weight, based on the weight of the preparation, and preferably in the range of about 0,05% to 1%. In the case of sodium monofluorophosphate, the compound may be present in an amount of about 0.1-3%. In dentrifrice preparations such as lozenges and chewing gum, the fluorine-providing compound is typically present in an amount sufficient to release up to about 500 ppm, preferably about 25 to about 300 ppm by weight of fluoride ion. Generally about 0.005 to about 1.0 wt% of such compound is present.

Bovine colostrum, or extracts thereof in combination with fluoride are compatible to the usual mouth care products additives, such as abrasives, polishing agents, thickening agents, foaming agents, flavouring agents, sweeteners, preservatives and/or other basic ingredients.

The mouth-care product according to the present invention can have all suitable forms, like tooth paste or cream, dental gel, tooth powder, mouth-wash, chewing gum, dental or chewing tablet, lozenge or effervescent tablet or any other suitable form.

These products may contain the conventional components that are usually present in those products. The choice thereof depends on the actual type of product.

The mouth-care products according to the present invention can, if necessary, be based upon an orally acceptable vehicle.

In certain desirable forms of the present invention, the oral composition may be substantially solid or pasty in character, such as toothpowder, dental tablet, toothpaste or dental gel. Such solid or pasty oral preparations generally contains polishing material.

Examples of polishing materials are water-insoluble sodium metaphosphate, potassium metaphosphate, tricalcium phosphate, dehydrated calcium phosphate, anhydrous dicalcium phosphate, calcium pyrophosphate, magnesium orthophosphate, trimagnesium phosphate, aluminium oxide, aluminium oxide hydrate, calcium carbonate, aluminium silicate, zirconium silicate, silica, bentonite, and mixtures thereof. Other suitable polishing materials include the particulate thermosetting resins described in U.S. Patent No. 3 070 510, such as melamine-phenolic-, and urea-formaldehydes, and crosslinked polyepoxides and polyesters. Preferred polishing materials include silica having particle sizes of up to about 7 microns, a mean particle size of up to about 1.1 microns, and a surface area of up to about 50,000 cm2/gm, silica gel or colloidal silica, and complex amorphous alkali metal aluminosilicate.

When visually clear gels are desired, a polishing agent of colloidal silica, such as those sold under the trademark SYLOID as Syloid 72 and Syloid 74 or under the trademark SANTOCEL as Santocel 100 and alkali metal aluminosilicate complexes are particularly useful, since they have refractive indices close to the refractive indices of gelling agent-liquid (including water and/or humectant) systems commonly used in dentifrice's.

The polishing material is generally present in the solid or paste compositions in weight concentrations of about 10% to about 99%. Preferably, it is present in amounts ranging from about 10% to about 75% in toothpaste, and from about 70% to about 99% in toothpowder.

In a toothpaste, the liquid vehicle may comprise water and humectant typically in an amount ranging from about 10% to about 90% by weight of the preparation. Glycerine, propylene glycol, sorbitol, polypropylene glycol and/or polyethylene glycol exemplify suitable humectants/carriers. Also advantageous are liquid mixtures of water, glycerine and sorbitol. In clear gels where the refractive index is an important consideration, about 3-30 wt% of water, 0 to about 80 wt% of glycerine, and about 20-80 wt% of sorbitol is preferably employed.

Toothpaste's and gels typically contain a natural or synthetic thickener or gelling agent in proportions of about 0.1 to about 10, preferably about 0.5 to about 5 wt%. A suitable thickener is synthetic hectorite, a synthetic colloidal magnesium alkali metal silicate complex clay available for example as Laponite™ (e.g. CP, SP 2002, D) marketed by Laporte Industries Limited.

Other suitable thickeners include Irish moss, gum tragacanth, starch, polyvinylpyrrolidone, hydroxyethylpropyl cellulose, hydroxybutyl methyl cellulose, hydroxypropyl methyl cellulose, hydroxyethyl cellulose (e.g. available as Natrosol), sodium carboxymethyl cellulose, and colloidal silica such as finely ground Syloid™.

Any suitable flavouring or sweetening material may also be employed. Examples of suitable flavouring constituents are flavouring oils, e.g. oil of spearmint, peppermint, wintergreen, sassafras, clove, sage, eucalyptus, marjoram, cinnamon, lemon, and orange, and methyl salicylate. Suitable sweetening agents include sucrose, lactose, maltose, sorbitol, xylitol, sodium cyclamate, perillartine, APM (aspartyl phenyl alanine methyl ester), saccharine and the like. Suitably, flavour and sweetening agents may together comprise from about 0.1% to 5% or more of the preparation.

The bovine colostrum is compatible with various other materials, used as active components in oral care products, such as phosphates, such as trimetaphosphates and diammoniumphosphates, enzymes, anti-tartar agents such as pyrophosphates, zinc-compounds and phosphonates and/or other anti-plaque ingredients, such as triclosan, chlorhexidine, bromochlorophene and sanquinarine, agents for sensitive teeth, such as specific alkalimetal salts like strontium salts and/or potassium nitrate or - citrate, wound healing agents such as allantoin, chlorophyll, tocopherol and herbal extracts, activity enhancing agents, or boosters, as gantrez and some polymers, and specific ingredients as urea, xylitol, silicones, quaternary ammonium compounds and mixtures thereof. These components, as well as the amounts to be used, are known in the art. They are, where present, incorporated in the preparations in amounts which do not substantially adversely affect the properties and characteristics desired.

Examples of suitable additional anti-plaque agents are triclosan, chlorhexidine, bromochlorophene, sanguinarine, metal salts and xylitol. Anti-calculus agents can for example be chosen from pyrophosphate, zinc-compounds and diphosphonates.

Agents for sensitive dentition are usually specific akalimetal salts, like strontium salts and potassium nitrate, or citrate. Wound healing agents can for example be allantoin, chlorophyl, tocopherol and herbal extracts. Finally the activity enhancing agents, or boosters, can be present to enhance the delivery and retention of the active components. Examples are Gantrez® and some polymers.

The use of the bovine colostrum according to the present invention can advantageously be combined with the use of the enzyme systems that are disclosed in US patents Nos.4 150 113 and 4 871 532. According to these patents hydrogen peroxide producing enzym systems have advantageous effects on the reduction of oral bacterial activity. The most effective enzyme is the oxidoreductase enzyme glucose oxidase. This enzyme may be present in combination with other enzymes such as amyloglucosidase, dextranase and/or mutanase, optionally in the presence of zinc ion providing compounds and/or 8-hydroxyquinoline derivatives. Other enzymes like those disclosed in US patent 4 152 418 may also be present.

Such combination of enzymes and bovine colostrum has a combined advantageous effect on the oral cavity condition, i.e., it will lead to a reduction of the bacterial activity to diminish the deposition of dental plaque and calculus formation.

Other enzymes like lactoperoxidase, lactoferrin and lysozyme may also be present and provide an oral care product having advantageous properties.

A preferred embodiment of the mouth-care product according to the present invention is a mouth-care product containing an effective amount of nisin as well as an effective amount of the said oxidoreductase enzyme system.

Another preferred embodiment of the mouth-care product according to the present invention is a tooth paste containing an effective amount of nisin, as well as an effective amount of fluoride.

The vehicle or carrier in a tablet or lozenge is a non-cariogenic solid water soluble polyhydric alcohol (polyol) such as mannitol, xylitol, sorbitol, maltitol, a hydrogenated starch hydrolysate, Lycasin, hydrogenated glucose, hydrogenated disaccharide's, and hydrogenated polysaccharides, in an amount of about 90-98% by weight of the total composition. Solid salts such as sodium carbonate, sodium chloride, potassium bicarbonate or potassium chloride may totally or partially replace the polyol carrier.

Tableting lubricants, in minor amounts of about 0.1 to 5% by weight, may be incorporated into the table or lozenge formulation to facilitate the preparation of both the tablets and lozenges. Suitable lubricants include vegetable oils such as coconut oil, magnesium stearate, aluminium stearate, talc, starch and carbowax.

The compositions of the present invention can be incorporated in lozenges, or in chewing gum or other products, e.g. by stirring into a warm gum base or coating the outer surface of a gum base, illustrative of which may be mentioned jelutone, rubber latex, vinylite resins, etc., desirable with conventional plasticizers or softeners, sugar or other sweeteners or carbohydrates such as glucose, sorbitol and the like.

Lozenge formulations may optionally contain about 2% gum as barrier agent to provide a shiny surface as opposed to a tablet which has a smooth finish. Suitable non-cariogenic gums include Kappa carrageenan, carboxymethyl cellulose, hydroxyethyl cellulose, Gantrez, and the like.

The lozenge or tablet may optionally be coated with a coating material such as waxes, shellac, carboxymethyl cellulose, polyethylene maleic anhydride copolymer or Kappacarrageenan to further increase the time it takes the tablet or lozenge to dissolve in the mouth. The uncoated tablet or lozenge is slow dissolving, providing a sustained release rate of the lantibiotic agent. Accordingly, the solid dose tablet and lozenge compositions of the present invention affords a relatively longer time period of contact of the teeth in the oral cavity with the active ingredient.

The mouth-care product according to the present invention can be prepared by the conventional ways of preparing said mouth-care products. The lantibiotics can be added to the finished mouth-care product or can be added during the production process. The process parameters like temperatures and pressures may have the conventional values.

The pH of the dentrifice preparation of the invention is generally in the range of from about 4.5 to about 10 and typically from about 5.5 to 9. Surprisingly the combination of fluoride and lantibiotic results in an improved anti-bacterial activity over a broader range of pH.

The pH is preferably in the range of from about 5.5 to about 8.0. It is noteworthy that the compositions of the invention may be applied orally at the said pH ranges without substantially decalcifying or otherwise damaging dental enamel. The pH can be controlled with acid (e.g. citric acid or benzoic acid) or base (e.g. sodium hydroxide) or be buffered (e.g. with sodium citrate, benzoate, carbonate, or bicarbonate, disodium hydrogen phosphate, or sodium dihydrogen phosphate).

The invention will now be illustrated by means of the following examples.

### Example 1

### Tooth paste

A tooth paste is prepared according to the method known in the art and consists of the following components:
(All amounts are percent by weight unless otherwise indicated)

| | |
|---|---|
| glycerol | 15.0 |
| aluminium oxide hydrate | 35.0 |
| ethoxylated fatty alcohol | 3.0 |
| carraghene | 1.75 |
| esters of para-hydroxy-benzoic acids (PHB) | 0.15 |
| aroma | 1.2 |
| sodium fluoride | 0.33 |
| dried bovine colostrum | 10 |
| water | to 100 % |

### Example 2

### Mouth-wash

A mouth-wash is prepared according to the method known in the art and consists of the following components:
(All amounts are percent by weight unless otherwise indicated)

| | |
|---|---|
| glycerol | 15.0 |
| xylitol | 15.0 |
| aroma | 0.05 |
| Tween® 80 | 2.0 |
| propyl para hydroxy benzoate | 0.1 |
| sodium citrate | 0.2 |
| sodium fluoride | 0.1 |
| dried bovine colostrum | 5,0 |
| water | to 100 % |

### Example 3

### Lozenge

A lozenge is prepared according to the method known in the art and consists of the following components:
(All amounts are in mg unless otherwise indicated)

| | |
|---|---|
| sorbitol | 1500 |
| encapsulated aroma | 10 |
| sodium stearate | 10 |
| phosphate buffer pH = 5.5 | 50 |
| poly vinyl pyrrolidone (PVP) | 20 |
| sodium fluoride | 1.0 |
| dried bovine colostrum | 1000 |

### Example 4

### Chewing tablet

A chewing tablet is prepared according to the method known in the art and consists of the following components:
(All amounts are in percent by weight unless otherwise indicated)

| | |
|---|---|
| gum base | 25 |
| sorbitol | 17.5 |
| mannitol | 5 |
| aroma | 0.5 |
| glycerol | 3.0 |
| xylitol | 10 |
| sodium fluoride | 0.01 |
| dried bovine colostrum | 5,0 |
| sorbitol | to 100 % |

### Example 5

### Effervescent tablet

An effervescent tablet is prepared according to the method known in the art and consists of the following components:
(All amounts are in mg unless otherwise indicated)

| | |
|---|---|
| sodium carbonate | 40 |
| citric acid | 20 |
| poly vinyl pyrrolidone (PVP) | 2 |
| sorbitol | 24 |
| encapsulated aroma | 2 |
| sodium fluoride | 10 |
| dried bovine colostrum | 20 |
| dissolve in: 15 ml water | |

### Example 6

### Tooth powder

A tooth powder is prepared according to the method known in the art and consists of the following components:
(All amounts are in percent by weight unless otherwise indicated)

| | |
|---|---|
| aroma | 2 |
| sodium saccharinate | 0.15 |
| detergent | 1 |
| sodium fluoride | 0.33 |
| dried bovine colostrum | 25 |
| calcium phosphate | to 100 % |

### Example 7

### Tooth paste

A tooth paste is prepared according to the method known in the art and consists of the following components:
(All amounts are in percent by weight unless otherwise indicated)

| | |
|---|---|
| sorbitol | 22 |
| silica | 22 |
| ethoxylated fatty alcohol | 3.0 |
| carraghene | 1.5 |
| sodium benzoate | 0.1 |
| aroma | 0.6 |
| sodium saccharinate | 0.1 |
| amyloglucosidase | 18 U/g |
| glucose oxidase | 6 U/g |
| mutanase | 16 U/g |
| zinc gluconate | 8·10⁻⁴ |
| 5 chloro-8-hydroxyquinoline | 1·10⁻³ |
| sodium fluoride | 0.33 |
| dried bovine colostrum | 15 |
| water | to 100 % |

## Claims

1. Use of dried or purified bovine colostrum, for the manufacture of a mouth-care product for the treatment of irritations and/or diseases of the oral cavity, wherein said irritations and/or diseases are associated with the accumulation of dental plaque.

2. Use according to any of the previous claims, wherein said mouth-care product further comprises a fluoride providing agent.

3. Use according to any of the previous claims, wherein said product further comprises one or more phosphates, enzymes, anti-tartar agents, agents for sensitive teeth, wound healing agents, activity enhancing agents or boosters, urea, xylitol, silicones, quaternary ammonium compounds and mixtures thereof.

4. Use according to any of the previous claims, wherein the amount of colostrum is between 0.1 and 50 wt.%, preferably between 0.5 and 25 wt.%, calculated on the dry weight of said product

5. Use according to any of the previous claims, wherein in said product abrasive agents, polishing agents, thickening agents, colouring agents, sweetening agents, flavouring agents, foaming agents, other bactericidal agents and/or other active components are also present.

6. Use according to any of the previous claims, wherein said product is in the form of a tooth paste or cream, dental gel, tooth powder, mouth-wash, chewing gum, dental or chewing tablet, lozenge or effervescent tablet.

7. Use according to claim 6, wherein said product is in the form of a tooth paste or gel and further comprises a humectant.

8. Use according to any of the previous claims, wherein said irritations and/or diseases of the oral cavity are caries, gingivitis and/or other periodontal diseases.

9. Use according to any of the previous claims, wherein said treatment is prophylactic.

## Patentansprüche

1. Verwendung von getrocknetem oder gereinigtem Rinder-Kolostrum für die Herstellung eines Mundpflegeproduktes für die Behandlung von Irritationen und/oder Erkrankungen der Mundhöhle, worin die genannten Irritationen und/oder Erkrankungen In Verbindung stehen mit der Akkumulierung von Zahnbelag.

2. Verwendung nach einem der vorstehenden Patentansprüche, worin das genannte Mundpflegeprodukt weiterhin ein Fluorld-Bereltstellungsmittel umfasst.

3. Verwendung nach einem der vorstehenden Patentansprüche, worin das genannte Produkt welterhin ein oder mehrere Phosphate, Enzyme, Anti-Zahnsteinmittel, Mittel für empfindliche Zähne, Wundhellmittel, aktivitätsvergrößernde Mittel oder Verstärker, Harnstoff, Xylitol, Silicone, quaternäre Ammoniumverbindungen und Mischungen davon umfasst.

4. Verwendung nach einem der vorstehenden Patentansprüche, worin die Menge des Kolostrums zwischen 0,1 und 50 Gew.-%, bevorzugt zwischen 0,5 und 25 Gew,-%, bezogen auf das Trockengewichts des genannten Produktes, beträgt.

5. Verwendung nach einem der vorstehenden Patentansprüche, worin in dem genannten Produkt Schleifmittel, Pollermittel, Verdickungsmittel, Färbemittel, Süßstoffe, Aromastoffe, Treibmittel, weltere bakterizide Mittel und/oder weitere aktive Komponenten ebenfalls vorhanden sind.

6. Verwendung nach einem der vorstehenden Patentansprüche, worin das genannte Produkt In der Form einer Zahnpaste oder -creme, eines Dentalgels, eines Zahnpulvers, einer Mundspülung, als Kaugummi, als Dental- oder Kau-Tablette, als Lutschtablette oder als Brausetablette vorllegt.

7. Verwendung nach Patentanspruch 6, worin das genannte Produkt in der Form einer Zahnpaste oder eines -gels vorliegt und weiterhin ein Befeuchtungsmittel umfasst.

8. Verwendung nach einem der vorstehenden Patentansprüche, worin die genannten Irritationen und/oder Erkrankungen der Mundhöhle Karies, Zahnfleischentzündungen und/oder Perlodontal-Erkrankungen sind.

9. Verwendung nach einem der vorstehenden Patentansprüche, worin die genannte Behandlung prophylaktisch erfolgt.

## Revendications

1. Utilisation de colostrum bovin séché ou purifié pour la production de produits d'hygiène buccodentaire pour le traitement d'irritations et/ou de maladies de la cavité buccale, où lesdites irritations et/ou maladies sont associées à l'accumulation de la plaque dentaire.

2. Utilisation selon l'une quelconques des revendications précédentes, où ledit produit d'hygiène buccodentaire comprend en plus un agent fournissant du fluor.

3. Utilisation selon l'une quelconque des revendications précédentes, où ledit produit comprend en plus un ou plusieurs phosphates, enzymes, agents contre la formation de tartre, agents pour dents sensibles, agents cicatrisants, agents augmentant l'activité ou agents renforçants, de l'urée, du xylitol, des silicones, des composés d'ammonium quaternaires et leurs mélanges.

4. Utilisation selon l'une quelconque des revendications précédentes, où la quantité de colostrum est comprise entre 0,1 et 50 % en poids, de préférence entre 0,5 et 25 % en poids, par rapport au poids sec desdits produits.

5. Utilisation selon l'une quelconque des revendications précédentes, où ledit produit renferme également des agents abrasifs, des agents de polissage, des agents épaississants, des agents colorants, des édulcorants, des agents aromatisants, des agents moussants, d'autres agents bactéricides et/ou d'autres composants actifs.

6. Utilisation selon l'une quelconque des revendications précédentes, où ledit produit se présente sous forme d'une pâte ou crème dentifrice, de gel dentaire, de poudre dentaire, de bain de bouche, de chewing-gum, de comprimés dentaires ou pastilles à mâcher, de pastilles ou comprimés effervescents.

7. Utilisation selon la revendication 6, où ledit produit se présente sous forme de pâte ou gel dentifrice et comprend en plus un humectant.

8. Utilisation selon l'une quelconque des revendications précédentes, où lesdites irritations et/ou maladies de la cavité buccale sont des caries, la gingivite et/ou des périodontites.

9. Utilisation selon l'une quelconque des revendications précédentes, où ledit traitement est préventif.
